# EUROPEAN PATENT APPLICATION

(11) **EP 3 195 791 A1**
(43) Date of publication of application: **26.07.2017**
(21) Application number: 16830077.0
(22) Date of filing: 26.01.2016
(51) Int. Cl.: A61B 1/04, H04N 5/225

(54) **IMAGE DATA TRANSMISSION SYSTEM**

(30) Priority: 24.07.2015 JP 2015147050
(71) Applicant: Olympus Corporation, Tokyo 192-8507 (JP)
(72) Inventor: SAITO, Saeri, Hachioji-shi Tokyo 192-8507 (JP)
(74) Representative: Gunzelmann, Rainer
(86) International application number: PCT/JP2016/052163
(87) International publication number: WO 2017/017972

(57) **Abstract**

In an image data transmission system, even when encoded serial digital data transmitted from an endoscope 2 to a video processor 3 cannot be decoded due to a shift in a delimiter position as a result of a lack of the serial digital data or the like, a communication transmission section 37 transmits error announcement information to a communication reception section 27 in the endoscope 2 based on a decoding error signal from a decoding error detection section 36, and a control code insertion section 25 that has received the information instructs an encoding section 23 to insert a control code for synchronization at a timing of occurrence of an error.

## Description

### Technical Field

The present invention relates to an image data transmission system, and particularly, to an image data transmission system configured to convert parallel digital data outputted from an image pickup section into serial digital data and transmit the serial digital data.

### Background Art

Conventionally, an endoscope including an image pickup device configured to observe a subject is widely used in a medical field and an industrial field. A technique configuring an endoscope system detachably connected to an endoscope is also known, wherein a signal processing apparatus called a video processor executes various types of signal processing regarding the endoscope.

As for the endoscope system described above, an endoscope system including an endoscope is proposed in recent years, wherein a CMOS image sensor is adopted as an image pickup device, for example. A CMOS image sensor adopted in this type of endoscope is proposed, the CMOS image sensor including: a sensor section (image pickup section) configured to photoelectrically convert light from an optical system and output an electrical signal as image information; an AFE section configured to perform noise removal and A/D conversion of the electrical signal outputted by the sensor section; and a P/S conversion section configured to perform parallel/serial conversion of a parallel digital signal outputted by the AFE section and transmit the signal as serial digital data to the outside (Japanese Patent Application Laid-Open Publication No. 2014-033788).

A high-speed serial transmission system that is so-called 8B10B is known as a system for transmitting the serial digital data as described above. The 8B10B transmission system is a system for converting 8-bit data to 10-bit data and transmitting the 10-bit data. More specifically, a set of 8-bit data is encoded into 10-bit word data, and the 10-bit word data is transmitted.

In the 8B10B encoded transmission system, a code for synchronization (K code) is also transmitted in addition to general video data (D code) in order to find a delimiter of the word data during decoding on a reception side.

That is, during the decoding on the reception side, the code for synchronization is first found, and the delimiter of the word data is found. The delimiter position is used to receive the data of the video.

On the other hand, in the endoscope system as described above, an electric scalpel or the like may be used to perform a treatment in parallel with observation and treatment of a subject by the endoscope. In this case, data of, for example, 1 bit may not be received during the transmission of the serial digital data due to an effect of disturbance noise caused by the electric scalpel or the like.

Similarly, extra reception of data of 1 bit (that is, a bit that does not exist) in the serial digital data may be recognized due to the effect of the disturbance noise, and the delimiter position of decoding may be shifted.

In such a case, the delimiter position is shifted on the reception side, and the video data cannot be correctly received. The decoding is continuously failed until the code for synchronization is received again to find the delimiter position again.

In this state, the image is not displayed on a monitor until the code for synchronization is received again, or an abnormal image is displayed. This means that an image with poor quality is displayed.

In other words, there may be a situation in which an in-vivo image of an object cannot be observed during the use of the electrical scalpel. Therefore, it is desirable to quickly recover the state of correctly transmitting the video data when a decoding error is detected.

The code for synchronization is generally embedded at the start and is also periodically embedded at a rate of, for example, once per 1V (1 vertical synchronization signal).

Therefore, the frequency of appearance of the code for synchronization can be increased to reduce the time period from the shift in the delimiter position to the correction of the delimiter position. However, the reduction in the time period may lead to a problem of a decrease in the amount of transmission of general image data, or there is an adverse effect that the transmission speed needs to be increased to ensure the necessary amount of transmission.

In view of the circumstances, a technique is illustrated in Japanese Patent No. 5548054, wherein an error correction is performed when there is a decoding error on the reception side of the serial data, and the data that cannot be corrected by the error correction is interpolated by surrounding pixels.

On the other hand, a technique is illustrated in U.S. Patent Application Publication No. 2013/0083178, wherein when the delimiter position of decoding is shifted, the general data (D code), instead of the code for synchronization, is used to search the delimiter position without waiting for arrival of the code for synchronization.

In the technique illustrated in Japanese Patent No. 5548054, the quality of the displayed image can be improved when decoding of one or a plurality data has failed due to garbled transmission data. However, when 1 bit is not received due to external noise or the like, or when the delimiter position of decoding is shifted due to the recognition of excessive reception of 1 bit as described above, the object of reducing the recovery time period of data transfer cannot be achieved.

In the technique described in U.S. Patent Application Publication No. 2013/0083178, the time period before the correction of the delimiter position is shorter than in the system of waiting for a normal control code. However, there is a problem that the reception side requires a dedicated circuit for detecting the synchronization timing in the general data.

The present invention has been made in view of the problems, and an object of the present invention is to provide an image data transmission system that can recover data transmission in a shorter time period even if decoding is obstructed by a malfunction of encoded and transmitted serial digital data due to external noise or the like.

### Disclosure of Invention

### Means for Solving the Problem

An aspect of the present invention provides an image data transmission system including: an image data transmission apparatus including: an encoding section configured to apply a predetermined encoding process to parallel digital data outputted from an image pickup section configured to pick up an image of an object to acquire image data and output the parallel digital data; a control code insertion section configured to issue an instruction for inserting a control code at a predetermined timing into the parallel digital data outputted from the image pickup section when the predetermined encoding process is applied by the encoding section; and a P/S conversion section configured to convert the parallel digital data subjected to the predetermined encoding process by the encoding section to serial digital data and transmit the serial digital data; and an image data reception apparatus including: an S/P conversion section configured to receive the serial digital data transmitted from the image data transmission apparatus to convert the serial digital data to parallel digital data and output the parallel digital data; a control code detection section configured to detect the control code from the parallel digital data converted and outputted by the S/P conversion section; a decoding section configured to apply a predetermined decoding process to the parallel digital data converted and outputted by the S/P conversion section according to the control code detected by the control code detection section; a decoding error detection section capable of detecting a decoding error in the predetermined decoding process by the decoding section; and a communication transmission section configured to transmit announcement information regarding the detection of the decoding error to the image data transmission apparatus when the decoding error is detected by the decoding error detection section, wherein the image data transmission apparatus further includes a communication reception section configured to receive the announcement information from the communication transmission section, and the control code insertion section instructs the encoding section to insert the control code at a predetermined timing to the parallel digital data outputted from the image pickup section according to a timing of the reception of the announcement information when the announcement information is received by the communication reception section.

### Brief Description of the Drawings

Fig. 1 is an external perspective view showing a configuration of an endoscope system that is an image data transmission system according to a first embodiment of the present invention;
Fig. 2 is a block diagram showing a schematic configuration of the image data transmission system according to the first embodiment;
Fig. 3 is a timing chart showing a transmission state of a video signal without a decoding error in the image data transmission system according to the first embodiment;
Fig. 4 is a timing chart showing a transmission state of a video signal without using an insertion function of a control code according to the present invention when there is a decoding error in the image data transmission system according to the first embodiment;
Fig. 5 is a timing chart showing a situation of normal recovery of video signal transmission using the insertion function of the control code according to the present invention when there is a decoding error in the image data transmission system according to the first embodiment;
Fig. 6 is a diagram showing a data structure during normal transmission of serial digital data subjected to 8B10B encoding in the image data transmission system according to the first embodiment;
Fig. 7 is a diagram showing an example of a data structure during an error in the serial digital data subjected to 8B10B encoding in the image data transmission system according to the first embodiment;
Fig. 8 is a timing chart showing a transmission state of a video signal when there is an error during a blanking period in the image data transmission system according to a first modification of the first embodiment;
Fig. 9 is a timing chart showing a transmission state of a video signal when an insertion timing of the control code and a start of a next blanking period are close to each other in the image data transmission system according to a second modification of the first embodiment;
Fig. 10 is a block diagram showing a schematic configuration of an image data transmission system according to a second embodiment of the present invention;
Fig. 11 is a block diagram showing a schematic configuration of an image data transmission system according to a third embodiment of the present invention;
Fig. 12 is a block diagram showing a schematic configuration of an image data transmission system according to a fourth embodiment of the present invention; and
Fig. 13 is a timing chart showing a signal state of each circuit in the image data transmission system according to the fourth embodiment.

### Best Mode for Carrying Out the Invention

Hereinafter, embodiments of the present invention will be described with reference to the drawings.

The invention is not limited by the embodiments. Furthermore, the same reference signs are provided to the same parts in the description of the drawings.

### <First Embodiment>

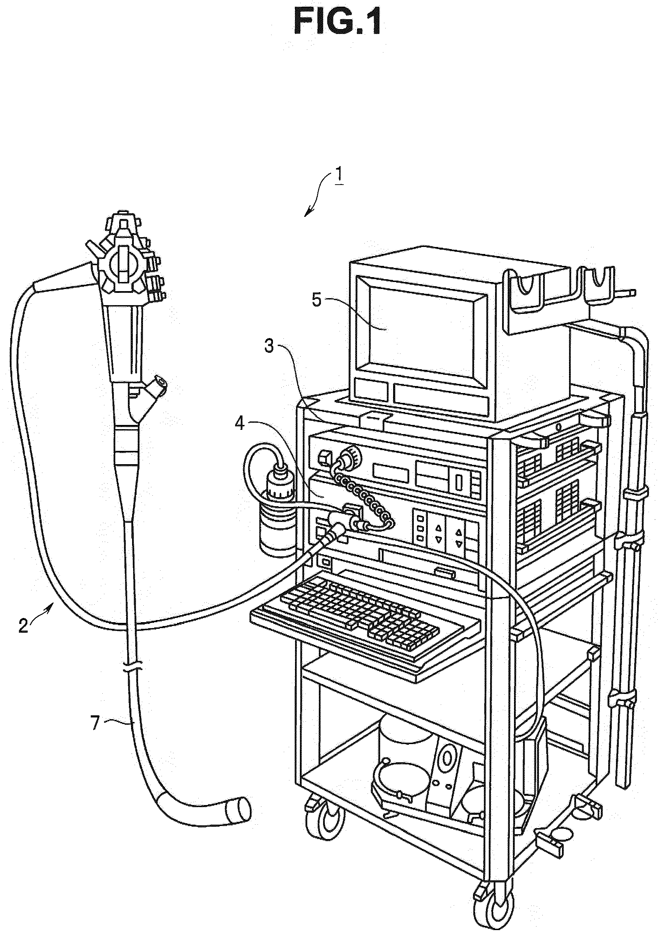

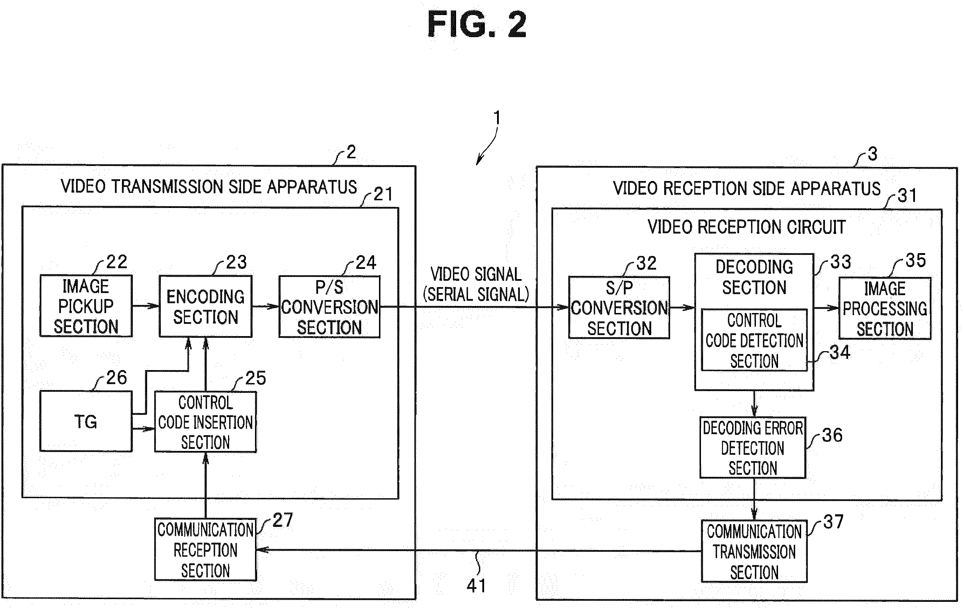

Fig. 1 is an external perspective view showing a configuration of an endoscope system that is an image data transmission system according to a first embodiment of the present invention. Fig. 2 is a block diagram showing a schematic configuration of an image data transmission system (endoscope system) according to the first embodiment of the present invention.

Note that in the embodiment illustrated below, an endoscope system will be described as an example of an "image data transmission system". An endoscope will be described as an example of a "video transmission side apparatus", and a video processor will be described as an example of a "video reception side apparatus".

As shown in Figs. 1 and 2, an endoscope system 1 that is an image data transmission system mainly includes: an endoscope 2 in which a distal end portion of an insertion portion 7 is inserted into a body cavity of a subject, thereby picking up an in-vivo image of an object and outputting a video signal of the object image as serial digital data; a video processor 3 configured to receive the video signal of the serial digital data outputted from the endoscope 2 to apply predetermined image processing to the video signal and configured to comprehensively control operation of the entire endoscope system 1; a light source apparatus 4 configured to generate illumination light to be emitted from a distal end of the endoscope 2; and a display apparatus 5 configured to display the image subjected to the image processing by the video processor 3.

The endoscope 2 includes an image pickup device 21 configured to receive the object image to photoelectrically convert the object image to an electrical signal and configured to apply predetermined signal processing.

The image pickup device 21 is configured by, for example, a CMOS image sensor and includes: a sensor section (image pickup section) 22 configured to photoelectrically convert light from an optical system and output an electrical signal as image information; an AFE section not shown configured to perform noise removal and A/D conversion of the electrical signal outputted by the image pickup section 22; an encoding section 23 configured to encode a parallel digital signal outputted by the AFE section based on 8B10B; a P/S conversion section 24 configured to perform parallel/serial conversion of the parallel digital data encoded by the encoding section 23 and output serial digital data (serial signal) to the outside; a control code insertion section 25 configured to issue an instruction for inserting a code for synchronization (hereinafter, called a control code) into the parallel digital data encoded by the encoding 23 at a predetermined timing; a timing generator (TG) 26 configured to generate pulses of drive timing of the image pickup section 22 and various types of signal processing by the AFE section, the encoding section 23, the P/S conversion section 24, and the control code insertion section 25; and a control section not shown configured to control operation of the image pickup device 21.

The endoscope 2 further includes a communication reception section 27 configured to receive predetermined decoding error information announced from the video processor 3 side through a dedicated line 41 (as described later, the decoding error information transmitted through the dedicated line 41 also serves as an insertion instruction of the control code for the control code insertion section 25) and configured to notify the control code insertion section 25 of the reception of the information when the decoding error information is received.

The video processor 3 includes a video reception circuit 31 configured to receive the video signal of the serial digital data, which is outputted from the P/S conversion section 24 in the endoscope 2 and subjected to 8B10B encoding, and apply predetermined image processing or the like to the video signal.

The video reception circuit 31 includes: an S/P conversion section 32 configured to receive the video signal of the encoded serial digital data to perform serial/parallel conversion and output parallel digital data to a subsequent stage; a control code detection section 34 configured to detect the control code from the parallel digital data; a decoding section 33 including the control code detection section 34 and configured to apply a decoding process to the encoded parallel digital data based on the control code detected by the control code detection section 34; a decoding error detection section 36 configured to detect a decoding error when there is a decoding error at the application of the decoding process by the decoding section 33; and an image processing section 35 configured to receive the video signal subjected to the decoding process by the decoding section 33 to apply predetermined image processing to the video signal.

The video processor 3 further includes a communication transmission section 37 connected to the decoding error detection section 36 and configured to transmit information of the decoding error to the communication reception section 27 in the endoscope 2 when the decoding error is detected by the decoding error detection section 36.

In the present embodiment, the communication transmission section 37 and the communication reception section 27 are connected by the dedicated line 41, and the decoding error information is transmitted from the communication transmission section 37 to the communication reception section 27 and the control code insertion section 25 through the dedicated line 41.

Note that the communication transmission section 37 functions to announce the decoding error information to the communication reception section 27 in the endoscope 2.

### <Action of First Embodiment>

Next, action of the present first embodiment will be described.

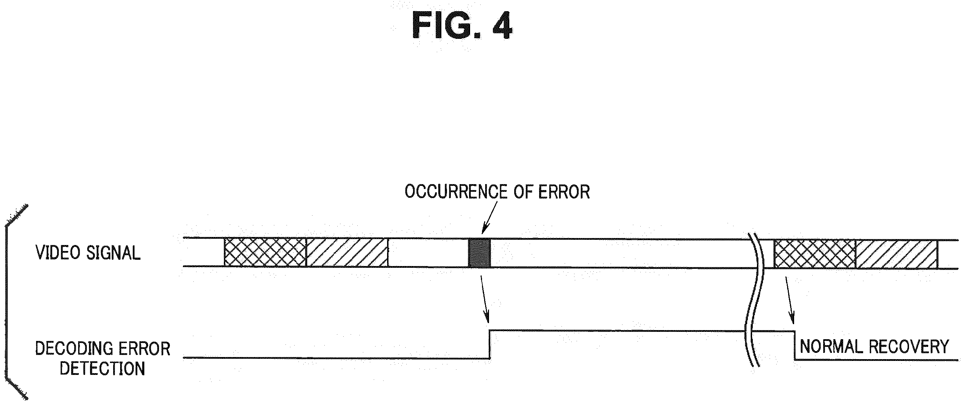

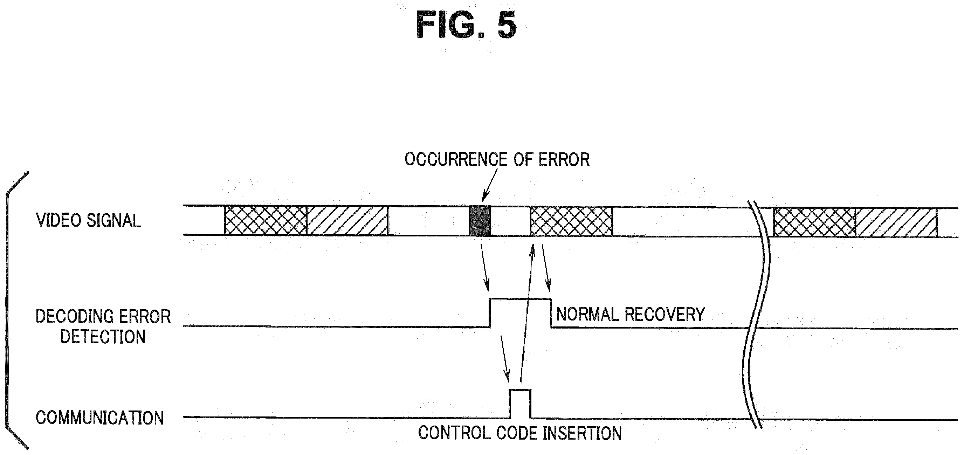

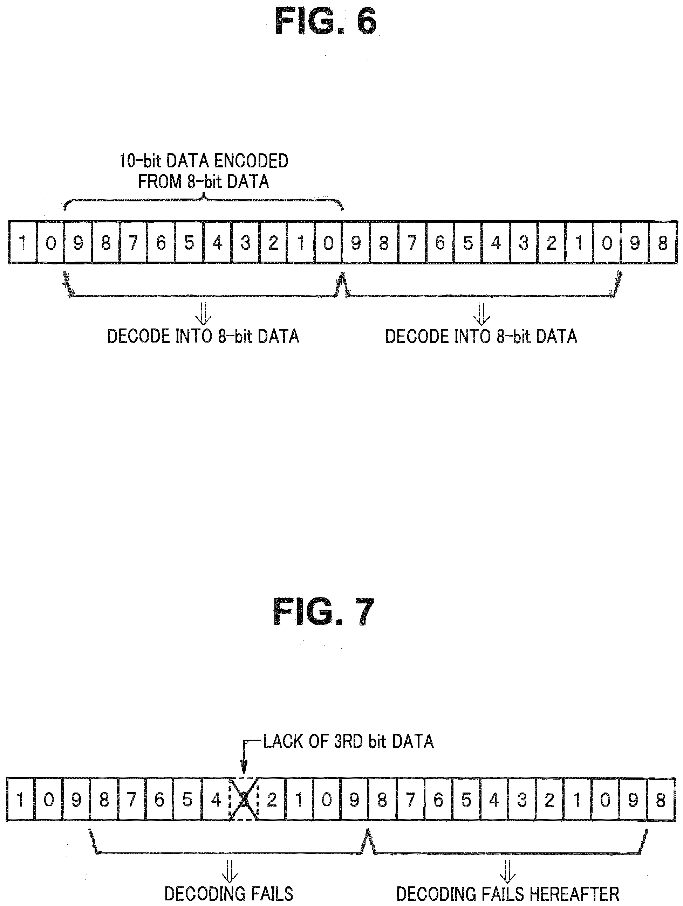

Fig. 4 is a timing chart showing a transmission state of the video signal when there is no decoding error in the image data transmission system according to the first embodiment. Fig. 5 is a timing chart showing a transmission state of the video signal without using the insertion function of the control code according to the present invention when there is a decoding error in the image data transmission system according to the first embodiment. Fig. 6 is a timing chart showing a situation of normal recovery of the video signal transmission using the insertion function of the control code according to the present invention when there is a decoding error in the image data transmission system according to the first embodiment.

### <A: "Normal State" without Decoding Error>

First, action in the normal state without a decoding error will be described.

In the endoscope 2, when the image pickup section 22 picks up an image of a subject, the image pickup section 22 outputs an analog electrical signal. Subsequently, the AFE section performs noise removal and A/D conversion of the electrical signal, and then the encoding section 23 encodes the parallel digital signal based on 8B10B. The P/S conversion section 24 performs parallel/serial conversion of the parallel digital data encoded by the encoding section 23 and outputs serial digital data to the outside.

Here, the encoding section 23 encodes a set of 8-bit data into 10-bit word data indicated by 0 to 9 as shown in Fig. 6 and transmits the 10-bit word data to a subsequent stage.

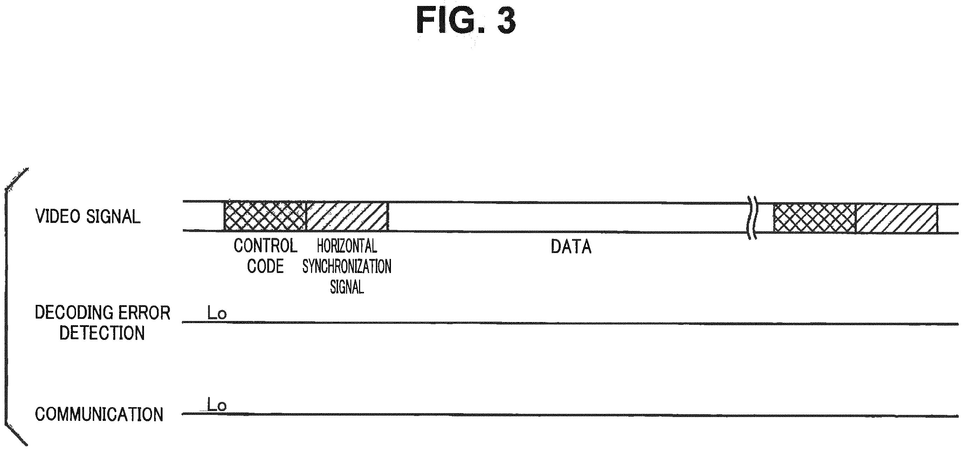

In this case, as shown in Fig. 3, when the encoding section 23 applies the encoding process to the parallel digital data, the encoding section 23 inserts the control code for synchronization into the video signal of the parallel digital data at a predetermined timing based on an instruction by the control code insertion section 25.

That is, the control code insertion section 25 instructs the encoding section 23 to insert the control code for synchronization according to the timing set by the timing generator 26. Note that Fig. 3 (and Figs. 4 and 5) illustrates an example of inserting one control code for each horizontal synchronization signal.

On the other hand, in the video reception circuit 31 of the video processor 3, the S/P conversion section 32 first receives the serial digital data outputted from the endoscope 2 side and then performs serial/parallel conversion of the received serial digital data.

Subsequently, the control code detection section 34 in the decoding section 33 detects the control code for synchronization from the parallel digital data subjected to the serial/parallel conversion by the S/P conversion section 32 and detects a delimiter position.

Subsequently, the decoding 33 applies a decoding process to the parallel digital data according to the delimiter position detected by the control code detection section 34.

In this case, the decoding section 33 decodes the 10-bit data encoded from the 8-bit data into 8-bit data as shown in Fig. 6.

Here, in the normal state without a decoding error, the decoding error detection section 36 does not detect a decoding error as shown in Fig. 4. Therefore, the communication transmission section 37 also outputs an "L level" signal indicating that there is no decoding error.

### <B: "Error State" with Decoding Error>

Next, action when there is a decoding error will be described.

In the endoscope 2, as described above, the electrical signal outputted from the image pickup section 22 goes through the AFE section, and the encoding 24 encodes the parallel digital signal based on 8B10B. The P/S conversion section 24 performs parallel/serial conversion and outputs serial digital data.

Subsequently, in the same way as described above, the encoding section 23 inserts the control code for synchronization into the video signal of the encoded parallel digital data according to the predetermined timing, that is, the timing set by the timing generator 26, under the instruction by the control code insertion section 25.

As shown for example in Fig. 7, it is assumed here that there is a lack of 1-bit data (3rd bit data in Fig. 7) in the encoded digital data due to external noise or the like.

On the other hand, in the video reception circuit 31 of the video processor 3, the control code detection section 34 tries to detect the control code for synchronization from the received encoded parallel digital data (parallel digital data through the S/P conversion section 32) to detect the delimiter position.

However, there is a lack of 1-bit data in the received parallel digital data (parallel digital data through the S/P conversion section 32) as described above, and the control code detection section 34 recognizes the delimiter position of the received digital data as a position shifted by 1 bit.

In response to the action of the control code detection section 34, the decoding 33 tries to decode the digital data based on the delimiter position shifted by 1 bit. However, the delimiter position is wrong as described above, and the decoding fails.

On the other hand, in response to the failure of decoding, the decoding error detection section 36 determines that there is a decoding error and outputs an "H level" signal indicating the detection of the decoding error to the communication transmission section 37 as shown in Fig. 4 or 5.

Note that the decoding error detection section 36 may determine that there is a decoding error in response to one failure in decoding as described above. However, whether the data is just garbled or the delimiter position is shifted may be difficult to determine based on just one error. Therefore, the decoding error detection section 36 may determine that the delimiter position is shifted when decoding errors occur for a plurality of times in a row.

Here, a case of not using the insertion function of the control code according to the present invention when there is a decoding error will be described.

Fig. 5 is a timing chart showing a transmission state of the video signal without using the insertion function of the control code according to the present invention when there is a decoding error in the present embodiment.

In this case, the decoding error detection section 36 transmits only the "H level" signal indicating the detection of the decoding error as shown in Fig. 5, and the control code insertion section 25 does not instruct insertion of the control code based on occurrence of a decoding error.

In this way, although the state with the occurrence of a decoding error can be recognized in this case (when the insertion function of the control code is not used), the recovery for inserting the control code at a next prescribed timing cannot be attained once there is a misalignment.

On the other hand, in the present invention, the communication transmission section 37 transmits the information of the decoding error to the communication reception section 27 in the endoscope 2 as described above when the decoding error detection section 36 detects a decoding error as shown in Fig. 5. In this way, the communication transmission section 37 instructs the control code insertion section 25 on the endoscope 2 side to insert the control code.

After receiving the decoding error information (which also serves as the insertion instruction of the control code for synchronization as described above), the communication reception section 27 sends out a notification indicating the reception of the instruction information to the control code insertion section 25.

After receiving the insertion instruction information of the control code for synchronization from the communication reception section 27, the control code insertion section 25 instructs the encoding 23 to insert the control code for synchronization into the video signal of the parallel digital data at the predetermined timing when the encoding process is applied to the parallel digital data.

Although the control code for synchronization inserted in this case is not based on the timing of a horizontal synchronization signal regarding the video signal, the alignment is normally recovered as a result of the insertion of the control code, and the video reception circuit 31 can then receive normal serial digital data within a short time period.

Note that a certain number of control codes for synchronization may be inserted, or the insertion may be continued until a notification of elimination of the decoding error is received from the video processor 3 side.

Although it is desirable to hold, in a predetermined memory or the like, the video signal intended to be transmitted during the insertion of the control code for synchronization and transmit the video signal again after the elimination of the decoding error, the video signal may be discarded.

Furthermore, there is usually a horizontal blanking period for not transmitting the video signal or a period for transmitting a control code in one horizontal synchronization signal in the transmission of the video signal. Therefore, the periods can be reduced in exchange for the additional insertion of the control code, and a lack of image can be reduced.

Although the communication transmission section 37 that has received the decoding error signal from the decoding error detection section 36 sends out the insertion instruction of the control signal for synchronization to the control code insertion section 25 in the present embodiment, the endoscope 2 side, such as the communication reception section 27 and the control code insertion section 25, may determine whether to insert the control signal for synchronization.

As described, in the present first embodiment, even when the serial digital data cannot be decoded due to the shift in the delimiter position of the serial digital data to be transmitted as a result of a lack of data or the like caused by disturbance noise or the like during the transmission of the serial digital data encoded based on 8B10B, the control code for synchronization is inserted into the serial digital data at the timing of the detection of the decoding error. The control can attain advantageous effects of reducing the time period for correcting the delimiter position and reducing the time period for recovering the video.

Next, a first modification of the present first embodiment will be described.

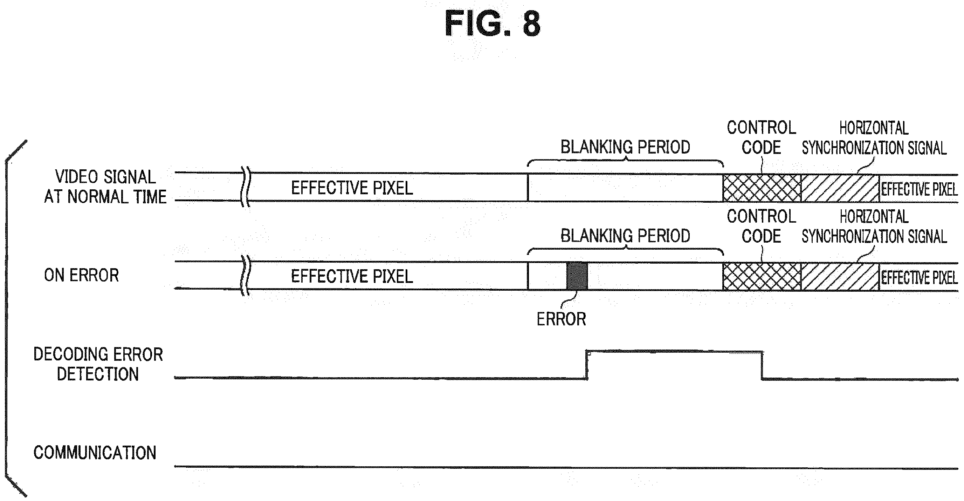

Fig. 8 is a timing chart showing a transmission state of the video signal when there is an error during the blanking period in the image data transmission system according to the first modification of the first embodiment.

In the first modification, the communication transmission section 37 just transmits an "L level" signal without doing anything when the timing of the detection of the decoding error by the decoding error detection section 36 is in the blanking period as shown in Fig. 8.

That is, even if there is an error during the blanking period, there is no real harm if the alignment is recovered by the next insertion of the control code (see Fig. 8). Therefore, the communication transmission section 37 is controlled to just transmit the "L level" signal as described above in the first modification.

Next, a second modification of the present first embodiment will be described.

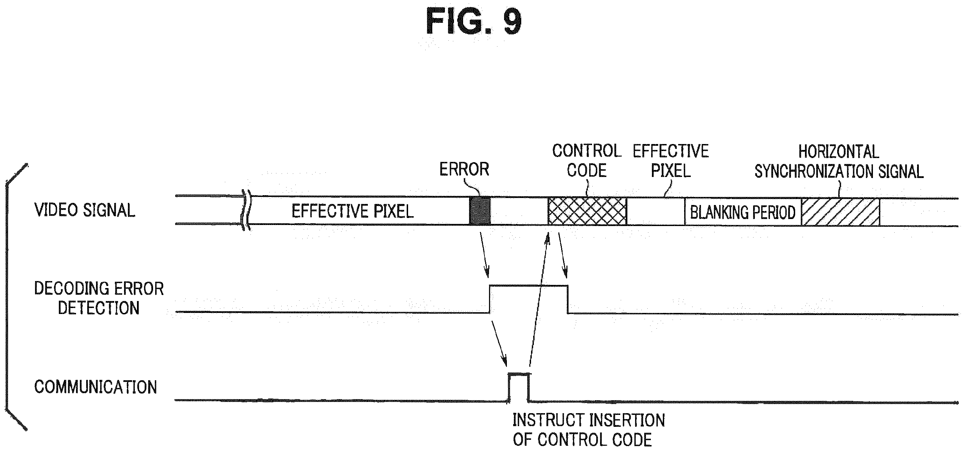

Fig. 9 is a timing chart showing a transmission state of the video signal when the insertion timing of the control code and the start of the next blanking period are close to each other in the image data transmission system according to the second modification of the first embodiment.

In the second modification, it is assumed that the control code insertion section 25 on the endoscope 2 side that has received the insertion instruction information of the control code from the communication transmission section 37 as a result of the detection of the decoding error instructs the encoding 23 to insert the control code for synchronization into the video signal of the parallel digital data at the predetermined timing, and the control code is actually inserted as in the first embodiment.

In this case, when the insertion timing of the control code and the start of the next blanking period are close to each other, that is, when the insertion timing of the control code is just before the start of the next blanking period, the insertion action is not performed for the periodically inserted control code at the original position as shown in Fig. 9 in the second modification.

### <Second Embodiment>

Next, a second embodiment of the present invention will be described.

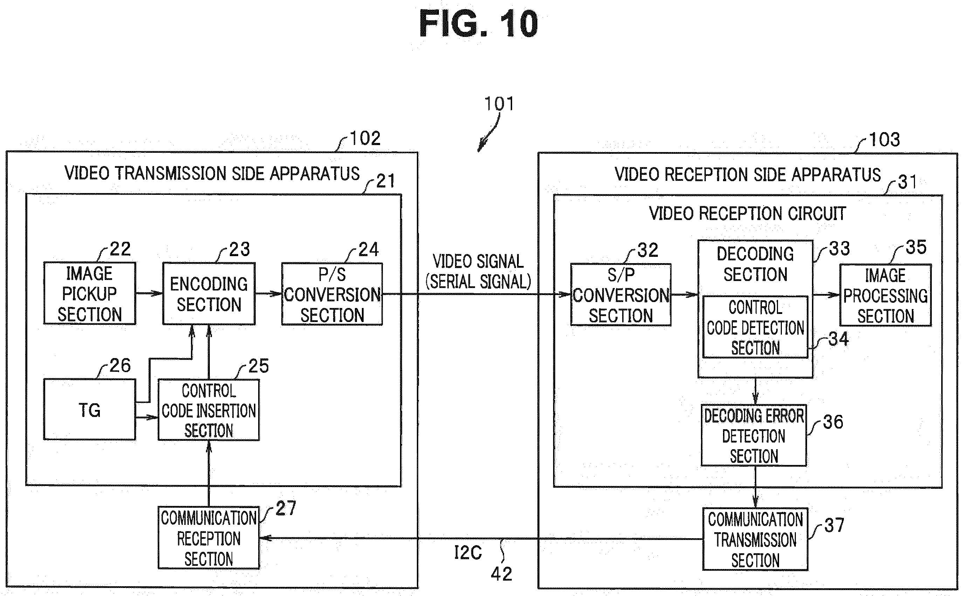

Fig. 10 is a block diagram showing a schematic configuration of an image data transmission system (endoscope system) according to the second embodiment of the present invention.

A basic configuration of an endoscope system 101 of the present second embodiment is the same as in the first embodiment, and only the configuration of transmitting the decoding error information from the communication transmission section 37 in a video processor (video reception side apparatus) 103 to the communication reception section 27 in an endoscope (video transmission side apparatus) 102 is different. Therefore, only the differences from the first embodiment will be described here, and the common parts will not be described.

As described, the communication transmission section 37 and the communication reception section 27 are connected by the dedicated line 41 (see Fig. 2) in the first embodiment, and the decoding error information is transmitted from the communication transmission section 37 to the communication reception section 27 and the control code insertion section 25 through the dedicated line 41.

In this way, the dedicated line is used for the insertion instruction (notification) of the control code from the video reception side apparatus to the video transmission side apparatus in the first embodiment. The dedicated line is not provided in the present second embodiment, and existing wiring is used to transmit the insertion instruction (notification) of the control code.

Here, as in the endoscope system 101 of the present embodiment, the video processor 103 as a video reception side apparatus and the endoscope 102 as a video transmission side apparatus are often connected by communication means.

In the present second embodiment, a CMOS image sensor is also adopted as an image pickup device in the endoscope 102 as in the first embodiment, and mode setting at the start or setting of a gain or the like of the video signal is transmitted by the communication means.

More specifically, the CMOS image sensor on the endoscope 102 side and the video reception circuit 31 on the video processor 103 side are connected by communication based on a clock synchronous serial communication standard that is so-called I2C (inter-integrated circuit) (see I2C control line 42 in Fig. 10).

By the way, the communication speed of the I2C communication is generally not so high. Therefore, if the I2C communication is used for the instruction of the insertion of the control code as described above as part of normal communication, a long time period is necessary for the completion of the communication and the actual insertion of the control code. This reduces the advantageous effects of the present invention.

Therefore, although the I2C communication control line is used in the present second embodiment, a protocol different from normal communication is used to prevent the decrease in the speed.

More specifically, according to the I2C communication of the present second embodiment, the start of communication is instructed by a start condition in a normal communication protocol. Subsequently, a slave address of a communication destination is designated, and then the address and the data of communication are transferred.

More specifically, a dedicated protocol (rule) is set such that the "occurrence of the start condition twice in a row suggests the notification of the insertion of the control code".

As described, the same advantageous effects as in the first embodiment can be attained in the present second embodiment without using a dedicated line for instructing (notifying) the insertion of the control code.

Note that although the I2C (inter-integrated circuit) communication is adopted as the communication means in the present second embodiment, the communication means is not limited to this. The same advantageous effects as in the present second embodiment can also be attained by adopting other serial communication such as SPI communication.

### <Third Embodiment>

Next, a third embodiment of the present invention will be described.

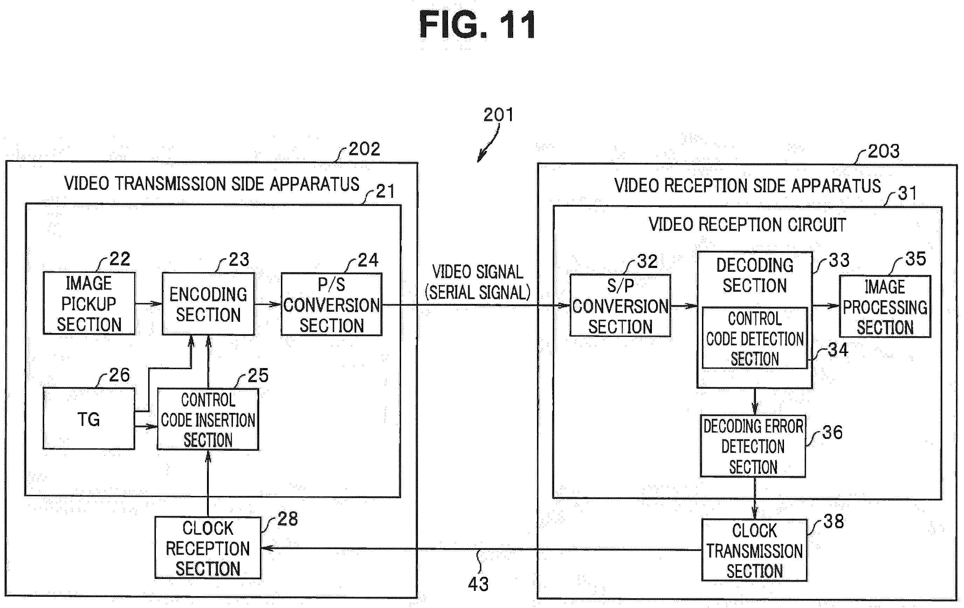

Fig. 11 is a block diagram showing a schematic configuration of an image data transmission system (endoscope system) according to a third embodiment of the present invention.

A basic configuration of an endoscope system 201 of the present third embodiment is the same as in the first and second embodiments, and only the configuration of transmitting the decoding error information from a video processor (video reception side apparatus) 203 to an endoscope (video transmission side apparatus 202) is different. Therefore, only differences from the first and second embodiments will be described here, and common parts will not be described.

As described, the communication transmission section 37 and the communication reception section 27 are connected by the dedicated line 41 (see Fig. 2) in the first embodiment, and the decoding error information is transmitted from the communication transmission section 37 to the communication reception section 27 and the control code insertion section 25 through the dedicated line 41.

In this way, the dedicated line is used for the insertion instruction (notification) of the control code from the video reception side apparatus to the video transmission side apparatus in the first embodiment. The present third embodiment focuses on the fact that a clock line for transmitting a clock is included between the video reception side apparatus and the video transmission side apparatus. The dedicated line is not provided, and the clock line is used to transmit the insertion instruction (notification) of the control code.

That is, a clock transmission section 38 is provided on the video processor 203, and a clock reception section 28 is provided on the endoscope 202 in the present third embodiment. Although the clock transmission section 38 and the clock reception section 28 are used for normal clock transmission and reception between the video processor 203 and the endoscope 202, a clock line 43 between the clock transmission section 38 and the clock reception section 28 is used for the insertion instruction (notification) of the control code in the present third embodiment.

More specifically, although a normal clock is transmitted to the clock line 43 with a constant frequency at the normal time, a plurality of clocks are partially omitted when the insertion of the control code is notified. The video processor 203 on the video transmission side detects the partial omission of the clocks and instructs (notifies) the insertion of the control code.

Here, when there is an abnormality in the video signal due to a disturbance or the like, an abnormality (partial omission) may also occur in the clock. In view of the situation, the abnormality is notified by partial omission of the clock in the present third embodiment, and when the abnormality occurs in both the clock and the video, the video transmission side (video processor 203 side) can instruct the insertion of the control code without waiting for the error detection by the video reception side (endoscope 202 side). Therefore, the time period before the recovery of normal transmission is short, and the insertion of the control code can be instructed even when there is an abnormality only in the video signal.

As described, the same advantageous effects as in the first embodiment can also be attained in the present third embodiment without using the dedicated line for instructing (notifying) the insertion of the control code.

The present invention is not limited to the embodiments, and various changes, modifications, and the like can be made without departing from the scope of the present invention.

Note that in the embodiments, the endoscope system is described as an example of the "image data transmission system", the endoscope is described as an example of the "video transmission side apparatus", and the video processor is described as an example of the "video reception side apparatus". However, the present invention is not limited to the examples. For example, the "image data transmission system" can be an endoscope, the "video transmission side apparatus" can be an image pickup section in the endoscope, and the "video reception side apparatus" can be a connector circuit in a connector section of the endoscope. Furthermore, the present invention can also be applied between, for example, FPGAs in a substrate of an endoscope or a video processor.

By the way, in a conventional endoscope system, an endoscope includes a memory storing correction data, and there is a well-known technique of transmitting the correction data to a processor to correct an image. A communication line (dedicated line) is generally used to transmit the correction data.

Although there is a well-known example in which serial transmission that is not a differential transmission system is used as communication means in this type of endoscope system, high-speed communication is difficult in this case (for example, transfer of 1 kByte in each vertical synchronization signal).

That is, even when the serial transmission that is not the differential transmission system is used, an amount of data necessary for the correction is conventionally small, which is about 16 kByte for example. The transfer time period is about 0.3 seconds, and this is not particularly a problem. However, when, for example, a 1-byte correction value is required for each pixel of 1M pixels, the total amount of data is 1 MByte. Transfer of 1-kByte data in each vertical synchronization signal takes about 16 seconds, and this is a problem.

When the number of pixels of a mounted image sensor is large as in an endoscope system of recent years, the transfer time period increases further. An increase in the transfer time period leads to an extension of the time period before the start of the correction, and this is not preferable for the image quality.

Here, to simply increase the amount of data that can be transmitted, differential transmission can be adopted in the communication line to handle high-speed communication. However, the differential transmission increases the number of signals, and this increases the number of pins of the connector. Furthermore, the signal quality needs to be ensured to handle a high-speed signal, and this increases the cost.

In view of the circumstances, the present applicant proposes the following example of configuration.

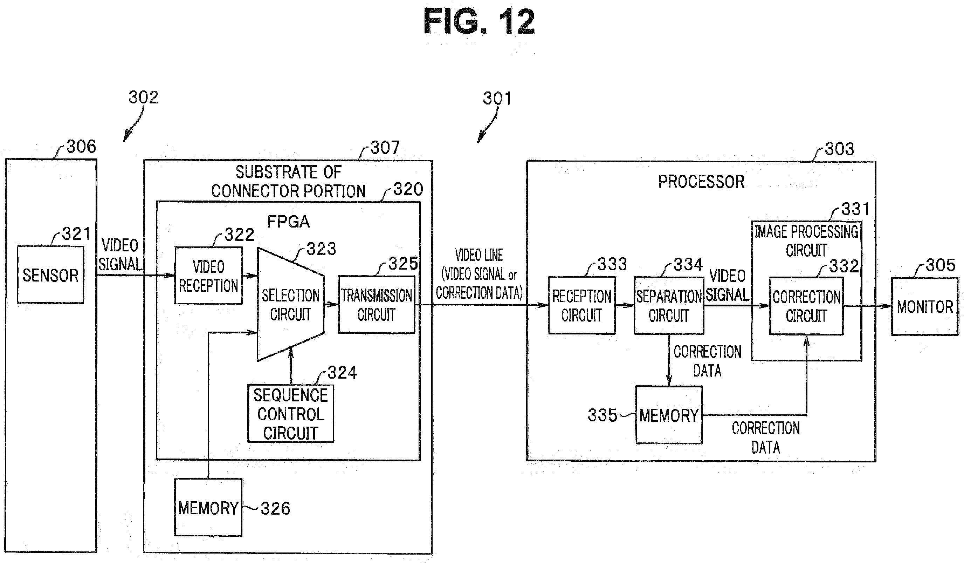

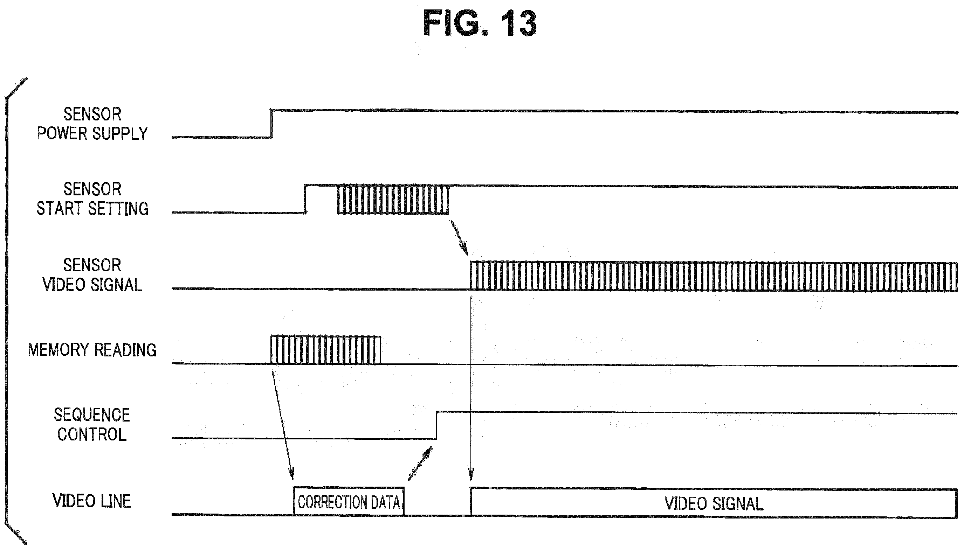

Fig. 12 is a block diagram showing a schematic configuration of an image data transmission system according to a fourth embodiment of the present invention. Fig. 13 is a timing chart showing a signal state of each circuit in the image data transmission system according to the fourth embodiment.

As shown in Fig. 12, an endoscope system 301 mainly includes: an endoscope 302 in which a distal end portion of an insertion portion is inserted into a body cavity of a subject, thereby picking up an in-vivo image of an object and outputting a video signal of the object image; a video processor 303 configured to receive the video signal or correction data outputted from the endoscope 302 through a video line to apply predetermined image processing to the video signal and configured to comprehensively control operation of the entire endoscope system 301; a light source apparatus (not shown) configured to generate illumination light to be emitted from a distal end of the endoscope 302; and a monitor 305 configured to display the image subjected to the image processing by the video processor 303.

The endoscope 302 includes: an image pickup device 306 located on the distal end portion of the insertion section, the image pickup device 306 configured to receive an object image to photoelectrically convert the object image into an electrical signal and configured to apply predetermined signal processing to output a video signal; and a connector portion 307 located on a proximal end side of a universal cord provided with various cables for connection with the video processor 303 and the light source apparatus.

The image pickup device 306 is configured by, for example, a CMOS image sensor and includes: a sensor 321 configured to photoelectrically convert light from an optical system and output an electrical signal as image information; an AFE section configured to perform noise removal and A/D conversion of the electrical signal outputted by the sensor 321; a timing generator configured to generate pulses of various types of signal processing; and a control section configured to control operation of the image pickup device 306 (the AFE section, the timing generator, and the control section are not shown).

The connector portion 307 includes: an FPGA (field programmable gate array) 320 configured to apply predetermined signal processing to the video signal outputted from the image pickup device 306; and a memory 326 configured to store correction data.

The FPGA 320 includes: a video reception section 322 configured to receive the video signal from the sensor 321; a selection circuit 323 configured to select and output the output (video signal) of the video reception section 322 and the correction data stored in the memory 326; a sequence control circuit 324 configured to control which signal is to be outputted from the selection circuit 323; and a transmission circuit 325 configured to transmit the signal (video signal or correction data) outputted from the selection circuit 323 controlled by the sequence control circuit 324 to the video processor 303 through a video line.

The sequence control circuit 324 is a control circuit configured to control the selection circuit 323 to switch a timing for sequentially transferring the correction data from the transmission circuit 325 through the video line and a timing for outputting the video signal through the video line.

On the other hand, the video processor 303 includes: a reception circuit 333 configured to receive the signal (video signal or correction data) outputted from the connector portion 307; a separation circuit 334 configured to separate the video signal or the correction data received by the reception circuit 333; a memory 335 configured to store the correction data separated by the separation circuit 334; and an image processing circuit 331 including a correction circuit 332 configured to use the correction data stored in the memory 335 to correct the video signal separated by the separation circuit 334.

### <Action>

Action of the present fourth embodiment configured as described above will be described with reference to Fig. 13.

First, the endoscope 302 starts to activate the sensor 321 in the image pickup device 306 when a sensor power supply not shown is turned on. Subsequently, the endoscope 302 starts to read the correction data stored in the memory 326 by the FPGA 320.

The sequence control circuit 324 then controls the selection circuit 323 to sequentially transfer the read correction data through the video line. Here, the selection circuit 323 selectively outputs the correction data stored in the memory 326, and the correction data is sequentially outputted from the transmission circuit 325 to the video line.

Subsequently, the sequence control circuit 324 controls the selection circuit 323 to output the video signal through the video line when the transfer of the correction data is completed. Here, the selection circuit 323 selectively outputs a video signal from the video reception section 322, and the video signal is outputted from the transmission circuit 325 to the video line.

On the other hand, when the reception circuit 333 receives the correction data and the video signal, the video processor 303 outputs the received signal to a subsequent stage, and the signal is separated into a video signal or correction data by the separation circuit 334.

Here, the correction data separated by the separation circuit 334 is temporarily stored in the memory 335 and outputted to the correction circuit 332 in the image processing circuit 331. Here, the correction circuit 332 uses the correction data stored in the memory 335 to correct the video signal separated by the separation circuit 334 and outputs the video signal to the monitor 305.

According to the configuration of the present fourth embodiment, when, for example, 1 Mbyte is transferred at 3 Gbps (in 8B10B encoding), the signal can be transferred in a shorter time period of about 0.03 seconds, which is faster than in the conventional example.

That is, according to the configuration of the present fourth embodiment, a large amount of correction data can be transmitted from the endoscope side to the video processor side in a short time period without additional cost for the transmission line.

The present invention can provide an image data transmission system that can recover data transmission in a shorter time period even if decoding is obstructed by a malfunction of encoded and transmitted serial digital data due to external noise or the like.

The present invention is not limited to the embodiments, and various changes, modifications, and the like can be made without departing from the scope of the present invention.

The present application is filed on the basis of claiming the benefit of priority from Japanese Patent Application No. 2015-147050, filed on July 24, 2015 in Japan, and the disclosed content is incorporated in the present specification and claims by reference.

## Claims

1. An image data transmission system comprising:
an image data transmission apparatus comprising:
an encoding section configured to apply a predetermined encoding process to parallel digital data outputted from an image pickup section configured to pick up an image of an object to acquire image data and output the parallel digital data;
a control code insertion section configured to issue an instruction for inserting a control code at a predetermined timing into the parallel digital data outputted from the image pickup section when the predetermined encoding process is applied by the encoding section; and
a P/S conversion section configured to convert the parallel digital data subjected to the predetermined encoding process by the encoding section to serial digital data and transmit the serial digital data; and
an image data reception apparatus comprising:
an S/P conversion section configured to receive the serial digital data transmitted from the image data transmission apparatus to convert the serial digital data to parallel digital data and output the parallel digital data;
a control code detection section configured to detect the control code from the parallel digital data converted and outputted by the S/P conversion section;
a decoding section configured to apply a predetermined decoding process to the parallel digital data converted and outputted by the S/P conversion section according to the control code detected by the control code detection section;
a decoding error detection section capable of detecting a decoding error in the predetermined decoding process by the decoding section; and
a communication transmission section configured to transmit announcement information regarding the detection of the decoding error to the image data transmission apparatus when the decoding error is detected by the decoding error detection section, wherein
the image data transmission apparatus further comprises a communication reception section configured to receive the announcement information from the communication transmission section, and
the control code insertion section instructs the encoding section to insert the control code at a predetermined timing to the parallel digital data outputted from the image pickup section according to a timing of the reception of the announcement information when the announcement information is received by the communication reception section.

2. The image data transmission system according to claim 1, further comprising
a dedicated communication line connecting the communication transmission section and the communication reception section.

3. The image data transmission system according to claim 1, wherein
a dedicated protocol is used for communication of the communication transmission section and the communication reception section.

4. The image data transmission system according to claim 1, wherein
a clock line connecting the image data transmission apparatus and the image data reception apparatus is used for communication of the communication transmission section and the communication reception section.
